# FASCICULE DE BREVET EUROPEEN

(11) **EP 1 994 882 B1**
(45) Date de publication et mention de la délivrance du brevet: **22.09.2010**
(21) Numéro de dépôt: 08156807.3
(22) Date de dépôt: 23.05.2008
(51) Int. Cl.: A61B 5/103, A61B 19/00

(54) **Sonde optique per-opératoire bi-spectrale**
Bispektrale, präoperativ eingesetzte optische Sonde
Bi-spectral peroperative optical probe

(30) Priorité: 25.05.2007 FR 0703738
(43) Date de publication de la demande: 26.11.2008
(73) Titulaire: Commissariat à l'Énergie Atomique et aux Énergies Alternatives, 75015 Paris (FR)
(72) Inventeur: Boutet, Jérôme, 38000 Grenoble (FR); Peltie, Philippe, 38760 Saint Paul de Varces (FR)
(74) Mandataire: Bréda, Jean-Marc

(56) Documents cités:
- DE-A1-102005 019 143

## Description

Le domaine de l'invention est celui des sondes optiques per-opératoires destinées à aider le chirurgien dans son geste médical.

Les chirurgiens utilisent des sondes optiques positionnables au-dessus d'un patient. Les sondes optiques fonctionnent en éclairage visible pour éclairer les tissus biologiques sur lesquels les chirurgiens sont en train d'opérer. Ces sondes sont notamment utilisées pour la détection et l'élimination de certaines tumeurs. Ces sondes optiques jouent le rôle d'un microscope et elles sont classiquement reliées à un ordinateur permettant d'afficher les tissus biologiques éclairés par la sonde. A partir d'une simple image agrandie du tissu biologique, l'expérience du chirurgien est plus que nécessaire pour déterminer la différence entre un tissu sain et un tissu atteint. Lorsque les différences visuelles ne sont pas suffisamment apparentes, le chirurgien procède par biopsie sur les zones qu'il juge suspectes. Si celle-ci est positive, le chirurgien élimine une couche de tissus puis prélève à nouveau des cellules, refait une biopsie et recommence l'opération jusqu'à ce que la biopsie soit négative. Cette opération prend du temps et demeure parfois incomplète, certaines zones atteintes étant invisibles à l'oeil nu ou insuffisamment différentiées pour être reconnues par le chirurgien.

Pour améliorer ces dispositifs et faciliter la détection de zones malades, il est possible d'utiliser des marqueurs fluorescents. Ces marqueurs sont utilisés dans le domaine des analyses génétiques et immunologiques in vitro. Le principe général consiste à marquer une molécule appelée sonde avec un marqueur fluorescent. En présence de la molécule cible, cette sonde s'associe à celle-ci. La fluorescence est mesurée en excitant le marqueur à l'aide d'un faisceau de lumière possédant une longueur d'onde adaptée. Suite à cette excitation, le fluorophore se relaxe en émettant une lumière à une longueur d'onde supérieure à la longueur d'onde d'excitation. Au moyen de filtres spécifiques, il est possible de détecter, de localiser et de quantifier cette fluorescence et ainsi de déterminer la présence de la molécule cible.

L'application de la fluorescence à l'imagerie in-vivo est assez récente. En effet, les tissus vivants absorbent les longueurs d'onde visibles généralement émises par fluorescence. Des progrès récents ont cependant permis d'appliquer cette technique aux tissus vivants. Ces progrès concernent essentiellement :
- L'évolution des sources de lumière vers les longueurs d'onde situées dans le rouge ou le proche infrarouge. Ces longueurs d'onde traversent mieux les tissus biologiques, l'hémoglobine étant l'absorbant principal de la lumière. Il est préférable que les longueurs d'onde ne soient pas supérieures à 900 nanomètres pour éviter l'absorption par l'eau ;
- L'augmentation de puissance des sources des systèmes d'éclairage. Ces sources sont le plus souvent des diodes électro-luminescentes ou des diodes laser ;
- L'amélioration de la sensibilité des photo-détecteurs.

Aussi, des systèmes d'imagerie de fluorescence in-vivo ont été développés. Ils sont plus particulièrement destinés aux petits animaux de laboratoire comme les souris pour le suivi de l'évolution de tumeurs ou pour étudier l'efficacité de médicaments.

Dans le domaine de l'humain, des prototypes de sonde utilisant la fluorescence ont été développés. Ces sondes comprennent essentiellement un laser et une caméra. On peut, par exemple, disposer la caméra au-dessus de la table d'opération. Dans ce cas, le champ de vision est très important et ne permet pas d'atteindre des résolutions élevées. On peut également utiliser des sondes de taille suffisamment petite pour tenir dans la main. Dans ce cas, la résolution est bien supérieure. Ainsi, une sonde ayant un champ de l'ordre de 8 centimètres et une distance de travail de l'ordre de 10 à 15 centimètres a une résolution inférieure à 100 microns.

Dans tous les cas, l'image fournie par la caméra est renvoyée vers un moniteur qui peut être un écran d'ordinateur. Le chirurgien voit ainsi une image en fluorescence et magnifiée de la zone éclairée par la sonde. Un dispositif de ce type est décrit dans le brevet français FR 2 882 147 intitulé « dispositif d'imagerie de fluorescence par réflexion à deux longueurs d'onde ». Ce brevet décrit un dispositif comportant une amélioration supplémentaire. La source d'excitation comporte deux longueurs d'onde d'excitation, permettant de s'affranchir de l'autofluorescence des tissus vivants.

Cependant, ces sondes à fluorescence comportent certains inconvénients. En effet, le chirurgien doit constamment porter son regard de son écran à la zone d'opération. De plus, ses instruments de chirurgie n'apparaissent pas en lumière fluorescente. Par conséquent, il ne peut faire correspondre précisément son geste chirurgical avec les cellules malades qu'il souhaite enlever. Le brevet DE 10 2005 019 143 décrit une sonde optique per-opératoire comportant des moyens de projection d'une image sur la zone à traiter.

Le but de l'invention est de pallier aux inconvénients précédents. A cette fin, les sondes optiques à fluorescence selon l'invention possèdent un double éclairage. Le premier est nécessaire pour réaliser la fluorescence des zones marquées et obtenir une image exploitable par une caméra. Le second est nécessaire pour éclairer ou pointer, en lumière visible, les zones marquées tout en laissant dans l'ombre les zones non marquées, facilitant ainsi le travail du chirurgien.

On obtient alors les avantages suivants :
- Le chirurgien voit directement les zones malades en éclairage visible ;
- Le confort visuel pour le chirurgien est accru. Il n'a plus besoin d'effectuer des aller-retour entre son écran d'ordinateur et le corps du patient,
- La précision du geste chirurgical est améliorée ;
- La durée de l'intervention chirurgicale est diminuée.

Plus précisément, l'invention a pour objet une sonde optique per-opératoire définie dans les revendications et comprenant au moins :
- une première source de lumière émettant un premier faisceau de lumière destiné à éclairer une surface de tissus biologiques dont au moins une zone dite zone marquée a été préalablement fixée par un marqueur fluorescent, ledit faisceau étant émis à une première longueur d'onde correspondant sensiblement à la longueur d'onde d'excitation du fluorophore de marquage, ledit fluorophore émettant à une seconde longueur d'onde ;

• un premier dispositif optique comprenant un premier objectif et une surface photo-sensible, ledit objectif formant de la surface de tissus biologiques une première image sur la dite surface photo-sensible, le premier dispositif étant relié à des moyens de traitement de l'image issue de la surface photo-sensible ;
• des moyens optiques différents du premier dispositif optique et destinés à éclairer au moins une partie de la surface de tissus biologiques.

Avantageusement, cette partie de surface éclairée par les moyens optiques correspond à au moins une des zones marquées identifiée par les moyens de traitement de l'image.

Les moyens optiques comprennent au moins un projecteur d'images générant, sur commande d'un dispositif de commande analysant le signal issu des moyens de traitement, une seconde image à au moins une troisième longueur d'onde située dans le spectre visible, ladite seconde image étant représentative de la zone marquée et un second objectif formant de cette seconde image une troisième image sur la surface de tissus biologiques, le second dispositif étant agencé de façon que cette troisième image visible soit de même dimensions et en coïncidence avec au moins une zone marquée de la surface de tissus biologiques.

Les moyens optiques peuvent comprendre une optique séparatrice permettant de séparer spectralement la seconde longueur d'onde de la troisième longueur d'onde, ladite optique étant disposée de façon que les parties des axes optiques du premier objectif et du second objectif situées entre la surface de tissus biologiques et la dite optique séparatrice soient communes.

Avantageusement, le premier dispositif optique comprend un premier filtre optique transparent à la seconde longueur d'onde et le projecteur d'images comprend un second filtre optique transparent à la troisième longueur d'onde.

Avantageusement, dans un second mode de réalisation, les moyens optiques comprennent au moins une troisième source de lumière émettant un troisième faisceau à la première longueur d'onde, agencée de façon à éclairer le centre de la surface de tissus biologiques de façon quasi-ponctuelle.

Dans ce cas, les moyens optiques peuvent comprendre une quatrième source de lumière émettant un quatrième faisceau à une quatrième longueur d'onde située dans le spectre visible, agencée de façon à éclairer le centre de la surface de tissus biologiques de façon quasi-ponctuelle en coïncidence avec le second faisceau.

Avantageusement, l'optique séparatrice permet de séparer spectralement la seconde longueur d'onde de la quatrième longueur d'onde, ladite optique étant disposée de façon que les parties des axes optiques du premier objectif et de la quatrième source situées entre la surface de tissus biologiques et la dite optique séparatrice soient communes.

Avantageusement, les moyens de traitement d'images comportent des moyens de reconnaissance des zones marquées et en ce que ladite sonde comprend des moyens électroniques permettant d'activer la quatrième source de lumière lorsqu'une zone marquée est éclairée par le troisième faisceau de lumière.

L'invention concerne plus généralement un appareillage médical destiné à être relié à une sonde possédant tout ou partie des moyens précédents et comprenant les moyens de traitement et le dispositif de commande de la quatrième source de lumière.

Avantageusement, l'appareillage médical comprend un dispositif d'affichage permettant de visualiser l'équivalent de la première image ou de la seconde image et comporte un éclairage de type scialytique émettant à des longueurs d'onde différentes d'au moins la première longueur d'onde.

L'invention sera mieux comprise et d'autres avantages apparaîtront à la lecture de la description qui va suivre donnée à titre non limitatif et grâce aux figures annexées parmi lesquelles :
La figure 1 représente un exemple qui n'appartient pas à l'invention revendiquée.
La figure 2 représente un premier mode de réalisation de l'invention.
La figure 3 représente un second mode de réalisation d'une sonde selon l'invention.
La figure 4 représente une variante de ce second mode de réalisation.
Sur ces différentes figures, les conventions suivantes ont été adoptées. Les flèches simples en traits minces représentent la direction des rayons lumineux et les traits pointillés les axes optiques des objectifs.

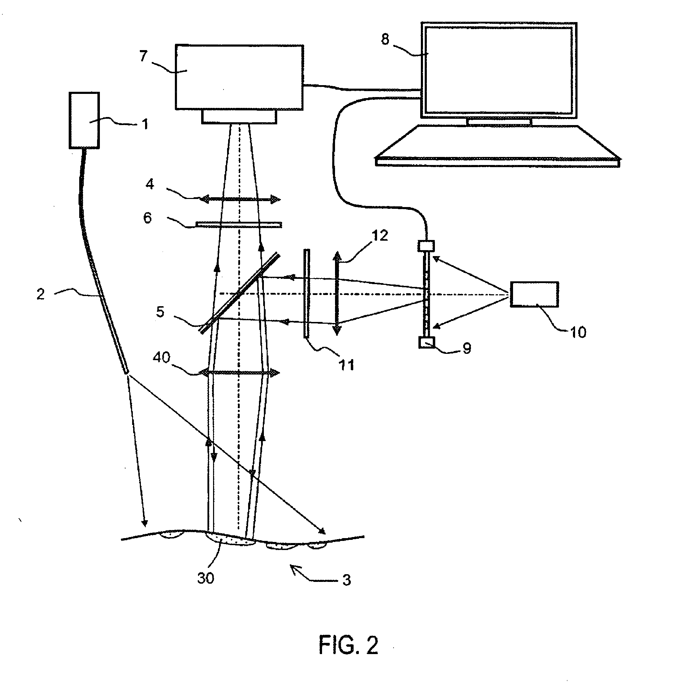

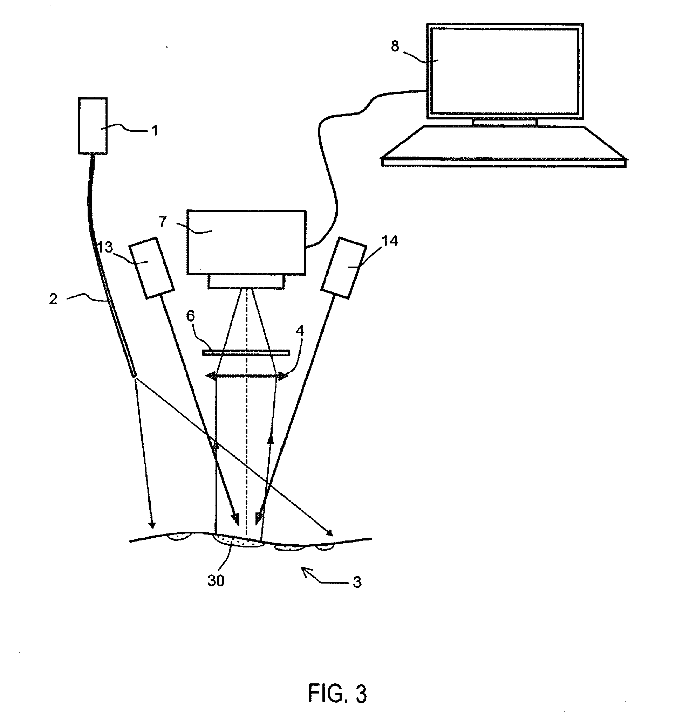

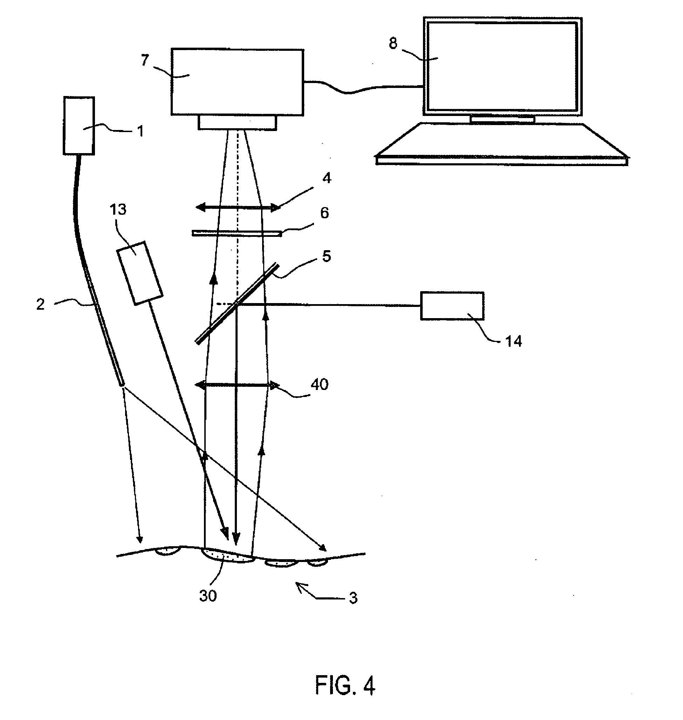

Il existe principalement deux modes de réalisation de sondes selon l'invention. Dans le premier mode de réalisation, on superpose à la surface de tissus biologiques que l'on cherche à opérer une image de la même zone en lumière visible. Dans un mode de réalisation plus simple, exclu de l'invention, on superpose à une zone marquée un éclairage quasi-ponctuel en lumière visible permettant de bien la localiser. Bien entendu, il est possible de coupler les deux dispositifs de façon à avoir à la fois un éclairage complet de la zone à opérer et un éclairage plus intense d'une zone particulière. A titre d'exemples non limitatifs, la figure 2 représente une variante du premier mode de réalisation et les figures 3 et 4 représentent deux variantes du second mode de réalisation.

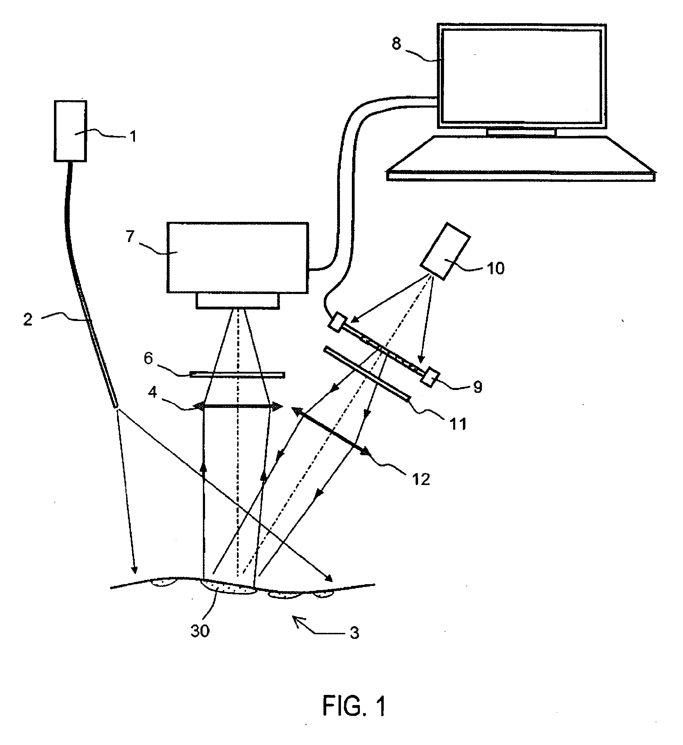

La figure 1 représente le schéma d'une sonde exclue de l'invention.

Une première source de lumière 1 émet un premier faisceau de lumière destiné à éclairer une surface de tissus biologiques 3 dont au moins une zone 30 dite zone marquée a été préalablement fixée par un marqueur fluorescent. Les techniques de marquage de tissus vivants sont bien connues par ailleurs et sortent du cadre de cette description. Le faisceau doit être émis à une première longueur d'onde correspondant sensiblement à la longueur d'onde d'excitation du fluorophore de marquage. Différentes sources permettent d'obtenir cet effet. Ainsi, la source peut être un laser, une diode électro-luminescente ou une lampe à incandescence. Elle peut comporter des dispositifs optiques annexes permettant de guider ou de diriger le faisceau lumineux afin d'améliorer sa répartition lumineuse sur la surface éclairée. Ainsi, à titre d'exemple, sur la figure 1, une fibre optique 2 permettant le transport de lumière de la source 1 à la zone éclairée 3 a été représentée.

Sous l'effet de la lumière d'excitation, le fluorophore émet à une seconde longueur d'onde. Un premier dispositif optique de type caméra comprenant un premier objectif 4 et une surface photo-sensible 7 forme de la surface de tissus biologiques 3 une première image sur la surface photo-sensible 7. Cette surface photo-sensible peut être de type CCD, par exemple. Ce premier dispositif peut comporter un filtre 6 dont la transmission est adaptée à cette seconde longueur d'onde. On évite ainsi toute lumière parasite issue soit de la première source, soit d'autres sources lumineuses disposées dans la salle d'opération.

Cette première image peut être traitée par des moyens de traitement de l'image appropriés permettant de mettre en évidence les zones marquées présentes dans l'image. Les traitements les plus simples sont les traitements d'image par seuillage d'intensité mais il est possible d'appliquer des traitements plus complexes. On citera, à titre d'exemple, les logiciels de traitement d'images Pro Plus ou Analysis.

Cette image traitée est transmise à un second dispositif optique comprenant au moins :
• un projecteur d'images 9 générant, à partir du signal issu des moyens de traitement, une seconde image à au moins une troisième longueur d'onde située dans le spectre visible, ladite seconde image étant représentative de la zone marquée. A titre d'exemple illustré en figure 1, on peut utiliser comme projecteur d'images une matrice d'affichage fonctionnant par transmission 9 éclairée par une source 10. Cette matrice peut être à cristaux liquides. On peut, bien entendu, utiliser des projecteur d'images fonctionnant par réflexion, comme par exemple une matrice de micro-miroirs. Il est également possible d'ajouter un filtre 11 dont la transmission soit adaptée à cette troisième longueur d'onde. Cette troisième longueur d'onde peut avantageusement être située dans le vert. Cette couleur présente le double avantage d'être située dans la zone de plus grande sensibilité de l'oeil humain et également d'assurer un excellent contraste avec la couleur rouge des tissus vivants.
• un second objectif 12 forme de cette seconde image une troisième image sur la surface de tissus biologiques 3, le second dispositif étant agencé de façon que cette troisième image visible soit de même dimensions et en coïncidence avec la surface de tissus biologiques.

Ainsi, les zones malades sont éclairées d'une lumière de couleur, facilitant le travail du chirurgien qui n'est pas obligé constamment de regarder un écran de visualisation.

L'ensemble constitué par la première source, les premiers et second dispositif optiques peut être monté dans une structure commune. Cette structure peut être agencée de façon à être facilement manipulable par l'opérateur. Dans ce cas, la sonde a un champ de l'ordre de 8 centimètres, une distance de travail de l'ordre de 10 à 15 centimètres et une résolution inférieure à 100 microns.

Dans l'exemple de la figure 1, le premier dispositif optique et le second dispositif ont des axes optiques disjoints. Cette disposition a deux inconvénients. D'une part, l'encombrement de la sonde est assez important. D'autre part, au moins un des axes optiques représentés en traits pointillés sur la figure 1 du premier ou du second dispositif optique n'est pas perpendiculaire au plan moyen de la surface de tissus biologiques, ce qui n'est pas favorable pour obtenir une image de bonne qualité, exempte d'aberrations optiques. Aussi, il est possible de pallier ces différents inconvénients en interposant une lame séparatrice qui va combiner la lumière de fluorescence issue des tissus vivants qui est à la seconde longueur d'onde avec la lumière issue du projecteur d'images qui est à une troisième longueur d'onde différente de la seconde longueur d'onde.

Une réalisation de ce type est illustré en figure 2. Cette sonde comprend les éléments décrits ci-dessous.

Une première source de lumière 1 émet un premier faisceau de lumière destiné à éclairer une surface de tissus biologiques 3 à une première longueur d'onde. Sous l'effet de la lumière d'excitation, le fluorophore émet à une seconde longueur d'onde.

Un premier dispositif optique de type caméra comprenant un premier objectif composé des ensembles optiques 4 et 40, une lame séparatrice 5 et une surface photo-sensible 7 forme de la surface de tissus biologiques 3 une première image sur la surface photo-sensible 7. A titre d'exemple, la lame séparatrice peut être un cube séparateur ou une lame dichroïque. Ce premier dispositif peut, de plus, comporter un filtre 6 dont la transmission est adaptée à cette seconde longueur d'onde. Sur la figure 2, la lame séparatrice est comprise entre deux ensembles optiques 4 et 40 et fonctionne en transmission. Bien entendu, il est possible d'adopter d'autres dispositions. Par exemple, on peut utiliser une seule optique disposée avant ou après la lame et la lame dichroïque peut fonctionner sur la voie caméra en transmission ou en réflexion.

Comme dans le cas de la figure 1, cette première image peut être traitée par des moyens de traitement de l'image appropriés permettant de mettre en évidence les zones marquées présentes dans l'image.

Cette image traitée est transmise à un second dispositif optique comprenant au moins :
• un projecteur d'images 9 générant, à partir du signal issu des moyens de traitement, une seconde image à au moins une troisième longueur d'onde située dans le spectre visible, ladite seconde image étant représentative de la zone marquée. Il est également possible d'ajouter un filtre 11 dont la transmission soit adaptée à cette troisième longueur d'onde.
• un second objectif 12 qui forme de cette seconde image, après réflexion sur la lame séparatrice 5 une troisième image sur la surface de tissus biologiques 3, le second dispositif étant agencé de façon que cette troisième image visible soit de même dimensions et en coïncidence avec la surface de tissus biologiques, les parties des axes optiques du premier objectif et du second objectif situées entre la surface de tissus biologiques et l'optique séparatrice 5 soient communes.

Lorsque le chirurgien bouge la sonde, la surface de tissus biologiques change. Il est très important que le délai entre le moment où l'image est captée par la caméra 7 et où elle est affichée par l'afficheur 9 soit suffisamment bref pour que l'image projetée ne présente pas de décalage perceptible avec la surface de tissus vivants.

Un second mode de réalisation d'une sonde selon l'invention est décrit sur les figures 3 et 4. La sonde de la figure 3 comprend essentiellement les éléments décrits ci-dessous :
Une première source de lumière 1 émettant un premier faisceau de lumière destiné à éclairer une surface de tissus biologiques 3 à une première longueur d'onde. Sous l'effet de la lumière d'excitation, le fluorophore émet à une seconde longueur d'onde.
Un premier dispositif optique de type caméra comprenant un premier objectif 4 et une surface photo-sensible 7 forme de la surface de tissus biologiques 3 une première image sur la surface photo-sensible 7. Ce premier dispositif peut comporter, comme dans les modes de réalisation précédents, un filtre 6.
Cette première image est traitée par des moyens de traitement de l'image appropriés 8 permettant de mettre en évidence les zones marquées présentes dans l'image et afficher sur un écran d'ordinateur ou un moniteur.
Un second dispositif optique comprenant au moins une seconde source de lumière 13 émettant un second faisceau à la première longueur d'onde, agencée de façon à éclairer le centre de la surface de tissus biologiques de façon quasi-ponctuelle.
Ce second dispositif peut comprendre une quatrième source de lumière 14 émettant un quatrième faisceau à une quatrième longueur d'onde située dans le spectre visible, agencée de façon à éclairer le centre de la surface de tissus biologiques de façon quasi-ponctuelle en coïncidence avec le troisième faisceau. Les troisième et quatrième sources peuvent être, par exemple, des lasers ou des diodes-lasers collimatées.
Ce second dispositif présente de nombreux avantages. D'une part, en positionnant les faisceaux des troisième et quatrième sources au centre du champ de la sonde, on est certain que la sonde est à la distance de travail permettant une netteté optimale sur l'écran de visualisation. D'autre part, dès que le chirurgien détecte sur son écran une zone marquée, il peut actionner les troisième et quatrième source et amener la zone détectée au centre du champ où elle est alors éclairée de façon optimale à la fois en éclairage fluorescent mais également en éclairage visible. On obtient une image de bonne qualité sur l'écran de visualisation et une image bien visible par le chirurgien qui opère.

Un perfectionnement intéressant de ce dispositif est que les moyens de traitement d'images comportent des moyens de reconnaissance des zones marquées et en ce que la sonde comprend des moyens permettant d'activer la quatrième source de lumière lorsqu'une zone marquée est éclairée par le troisième faisceau de lumière.

La sonde décrite en figure 4 constitue une variante de la précédente. Dans cette variante, on utilise comme déjà décrit sur la sonde de la figure 2 une lame séparatrice 5 permettant de combiner les faisceaux optiques issus des sources de lumière 13 et 14.

Il est possible de combiner les dispositifs décrits sur les figures 1 ou 2 avec ceux décrits sur les figures 3 ou 4 au prix d'adaptations optiques simples. On obtient alors une sonde qui peut, à la fois, projeter une image visible en coïncidence avec la surface de tissus biologiques et un spot lumineux éclairant la zone centrale des tissus. On réunit alors dans un même dispositif les différents avantages des réalisations précédentes.

La sonde peut être portée soit manuellement par l'opérateur, soit portée par un bras télescopique. La sonde fait partie d'un appareillage médical qui comporte généralement ses propres sources d'éclairage ambiant. On utilise généralement, à cet effet, des éclairages de type scialytique. Il est, dans ce cas, utile d'adapter ces sources d'éclairage de façon que leurs longueurs d'onde soient si possible différentes d'au moins la première longueur d'onde et de la seconde longueur d'onde qui correspond à la longueur d'onde de fluorescence des zones marquées. Ceci est en effet nécessaire afin d'éviter que la composante de la lumière émise par le scialytique correspondant à la longueur de fluorescence ne soit réfléchie par le patient et apparaisse sur la première image réalisée au niveau de la surface photo-sensible 7.

## Revendications

1. Sonde optique per-opératoire comprenant au moins :
une première source de lumière (1, 2) émettant un premier faisceau de lumière destiné à éclairer une surface (3) de tissus biologiques dont au moins une zone (30) dite zone marquée a été préalablement fixée par un marqueur fluorescent, ledit faisceau étant émis à une première longueur d'onde correspondant sensiblement à la longueur d'onde d'excitation du fluorophore de marquage, ledit fluorophore émettant à une seconde longueur d'onde ;
un premier dispositif optique comprenant un premier objectif (4) et une surface photo-sensible (7), ledit objectif formant de la surface de tissus biologiques une première image sur la dite surface photo-sensible, le premier dispositif étant relié à des moyens (8) de traitement de l'image issue de la surface photo-sensible ;
des moyens optiques différents du premier dispositif optique, comprenant un projecteur d'images (9, 10, 11) générant, sur commande d'un dispositif de commande analysant le signal issu des moyens de traitement, une seconde image à au moins une troisième longueur d'onde située dans le spectre visible, ladite seconde image étant représentative de la zone marquée;
un second objectif (12) formant de cette seconde image une troisième image sur la surface de tissus biologiques, le second dispositif étant agencé de façon que cette troisième image visible soit de même dimensions et en coïncidence avec au moins la zone marquée de la surface de tissus biologiques, identifiée par les moyens (8) de traitement de l'image; les moyens optiques étant **caractérisés par**
une optique séparatrice (5) permettant de séparer spectralement la seconde longueur d'onde de la troisième longueur d'onde, ladite optique (5) étant disposée de façon que les parties des axes optiques du premier objectif et du second objectif situées entre la surface de tissus biologiques et la dite optique séparatrice soient communes.

2. Sonde optique per-opératoire selon la revendication 1, **caractérisé en ce que** le premier dispositif optique comprend un premier filtre optique (6) transparent à la seconde longueur d'onde.

3. Sonde optique per-opératoire selon l'une des revendications 1 à 2, **caractérisé en ce que** le projecteur d'images (9, 10, 11) comprend un second filtre optique (11) transparent à la troisième longueur d'onde.

4. Sonde optique per-opératoire selon la revendication 1, **caractérisé en ce que** les moyens optiques comprennent au moins une troisième source de lumière (13) émettant un troisième faisceau à la première longueur d'onde, agencée de façon à éclairer le centre de la surface de tissus biologiques de façon quasi-ponctuelle.

5. Sonde optique per-opératoire selon la revendication 4, **caractérisé en ce que** les moyens optiques comprennent une quatrième source de lumière (14) émettant un quatrième faisceau à une quatrième longueur d'onde située dans le spectre visible, agencée de façon à éclairer le centre de la surface de tissus biologiques de façon quasi-ponctuelle en coïncidence avec le second faisceau.

6. Sonde optique per-opératoire selon la revendication 5, **caractérisé en ce que** l'optique séparatrice (5) sépare spectralement la seconde longueur d'onde de la quatrième longueur d'onde, ladite optique étant disposée de façon que les parties des axes optiques du premier objectif et de la quatrième source situées entre la surface de tissus biologiques et la dite optique séparatrice soient communes.

7. Sonde optique per-opératoire selon l'une des revendications 5 à 6, **caractérisé en ce que** les moyens de traitement d'images comportent des moyens de reconnaissance des zones marquées et **en ce que** ladite sonde comprend des moyens électroniques permettant d'activer la quatrième source de lumière lorsqu'une zone marquée est éclairée par le troisième faisceau de lumière.

8. Appareillage médical relié à une sonde selon l'une des revendications 5 à 7, comprenant lesdits moyens de traitement et le dispositif de commande de la quatrième source de lumière.

9. Appareillage médical selon la revendication 8, comprenant un dispositif d'affichage permettant de visualiser l'équivalent de la première image ou de la seconde image.

10. Appareillage médical selon l'une des revendications 8 ou 9, **caractérisé en ce qu'**il comporte un éclairage de type scialytique émettant à des longueurs d'onde différentes d'au moins la première longueur d'onde.

## Claims

1. An operative optical probe comprising at least:
a first light source (1, 2) emitting a first light beam for illuminating a biological tissue surface (3), at least one area (30) of which, referred to as the marked area, has been previously set by a fluorescent marker, said beam being emitted at a first wavelength substantially corresponding to the wavelength of excitation of the marking fluorophore, said fluorophore emitting at a second wavelength;
a first optical device including a first lens (4) and a photosensitive surface (7), said lens forming a first image of the biological tissue surface on said photosensitive surface, the first device being connected to means (8) for processing the image originating from the photosensitive surface;
optical means which differ from the first optical device, comprising an image projector (9, 10, 11) generating, as controlled by a control device analysing the signal originating from the processing means, a second image on at least a third wavelength located within the visible spectrum, said second image being representative of the marked area;
a second lens (12) forming a third image on the biological tissue surface from this second image, the second device being arranged so that this third visible image has the same dimensions and coincides with at least the marked area of the biological tissue surface, identified by the means (8) for processing the image; the optical means being **characterised by** an optical splitter (5) allowing the second wavelength to be divided spectrally from the third wavelength, said optical splitter (5) being arranged so that the parts of the optical axes of the first lens and the second lens which are located between the biological tissue surface and said optical splitter are shared.

2. The operative optical probe according to claim 1, **characterised in that** the first optical device includes a first optical filter (6) transparent to the second wavelength.

3. The operative optical probe according to any one of claims 1 to 2, **characterised in that** the image projector (9, 10, 11) includes a second optical filter (11) transparent to the third wavelength.

4. The operative optical probe according to claim 1, **characterised in that** the optical means include at least a third light source (13) emitting a third beam at the first wavelength, arranged so as to illuminate the centre of the biological tissue surface in a quasi-punctiform manner.

5. The operative optical probe according to claim 4, **characterised in that** the optical means include a fourth light source (14) emitting a fourth beam at a fourth wavelength located within the visible spectrum, arranged so as to illuminate the centre of the biological tissue surface in a quasi-punctifonn manner coinciding with the second beam.

6. The operative optical probe according to claim 5, **characterised in that** the optical splitter (5) spectrally splits the second wavelength from the fourth wavelength, said optical splitter being arranged so that the parts of the optical axes of the first lens and of the fourth source located between the biological tissue surface and said optical splitter are shared.

7. The operative optical probe according to any one of claims 5 to 6, **characterised in that** the image-processing means include means for identifying the marked areas, and **in that** said probe includes electronic means for activating the fourth light source when a marked area is illuminated by the third light beam.

8. A medical apparatus connected to a probe according to any one of claims 5 to 7, comprising said processing means and the control device of the fourth light source.

9. The medical apparatus according to claim 8, including a display device for visualising the equivalent of the first image or of the second image.

10. The medical apparatus according to any one of claims 8 to 9, **characterised in that** it comprises scialytic type lighting emitting at different wavelengths to at least the first wavelength.

## Patentansprüche

1. Intraoperative optische Sonde, die wenigstens Folgendes umfasst:
eine erste Lichtquelle (1, 2), die einen ersten Lichtstrahl zum Beleuchten einer biologischen Gewebefläche (3) emittiert, von der wenigstens eine Zone (30), markierte Zone genannt, zuvor mit einem Fluoreszenzmarker festgelegt wurde, wobei der Strahl mit einer ersten Wellenlänge emittiert wird, die im Wesentlichen der Erregungswellenlänge des Markierungsfluorophors entspricht, wobei das Fluorophor mit einer zweiten Wellenlänge emittiert;
eine erste optische Vorrichtung, die eine erste Linse (4) und eine lichtempfindliche Fläche (7) aufweist, wobei die Linse ein erstes Bild der biologischen Gewebefläche auf der lichtempfindlichen Fläche erzeugt, wobei die erste Vorrichtung mit Mitteln (8) zum Bearbeiten des von der lichtempfindlichen Fläche kommenden Bildes verbunden ist;
optische Mittel, die sich von der ersten optischen Vorrichtung unterscheiden und einen Bildprojektor (9, 10, 11) umfassen, der, gesteuert von einer Steuervorrichtung, die das von dem Bearbeitungsmittel kommende Signal analysiert, ein zweites Bild mit wenigstens einer dritten Wellenlänge erzeugt, die sich im sichtbaren Spektrum befindet, wobei das zweite Bild für die markierte Zone repräsentativ ist;
eine zweite Linse (12), die ein drittes Bild der biologischen Gewebefläche von diesem zweiten Bild erzeugt, wobei die zweite Vorrichtung so ausgelegt ist, dass dieses sichtbare Bild dieselben Abmessungen hat wie die markierte Zone der biologischen Gewebefläche und mit wenigstens dieser zusammenfällt, identifiziert durch die Bildverarbeitungsmittel (8); wobei die optischen Mittel **gekennzeichnet sind durch** einen optischen Teiler (5), mit dem die zweite Wellenlänge von der dritten Wellenlänge spektral getrennt werden kann, wobei der optische Teiler (5) so ausgelegt ist, dass die zwischen der biologischen Gewebefläche und dem optischen Teiler befindlichen Teile der optischen Achsen der ersten Linse und der zweiten Linse gemeinsame Teile sind.

2. Intraoperative optische Sonde nach Anspruch 1, **dadurch gekennzeichnet, dass** die erste optische Vorrichtung ein erstes optisches Filter (6) umfasst, das für die zweite Wellenlänge transparent ist.

3. Intraoperative optische Sonde nach Anspruch 1 oder 2, **dadurch gekennzeichnet, dass** der Bildprojektor (9, 10, 11) ein zweites optisches Filter (11) umfasst, das für die dritte Wellenlänge transparent ist.

4. Intraoperative optische Sonde nach Anspruch 1, **dadurch gekennzeichnet, dass** die optischen Mittel wenigstens eine dritte Lichtquelle (13) beinhalten, die einen dritten Strahl mit der ersten Wellenlänge emittiert und so ausgelegt ist, dass sie die Mitte der biologischen Gewebefläche quasi-punktuell beleuchtet.

5. Intraoperative optische Sonde nach Anspruch 4, **dadurch gekennzeichnet, dass** die optischen Mittel eine vierte Lichtquelle (13) beinhalten, die einen vierten Strahl mit einer im sichtbaren Spektrum liegenden vierten Wellenlänge emittiert und so ausgelegt ist, dass sie die Mitte der biologischen Gewebefläche quasi-punktuell zusammenfallend mit dem zweiten Strahl beleuchtet.

6. Intraoperative optische Sonde nach Anspruch 5, **dadurch gekennzeichnet, dass** der optische Teiler (5) die zweite Wellenlänge von der vierten Wellenlänge spektral trennt, wobei der optische Teiler so ausgelegt ist, dass die Teile der optischen Achsen der ersten Linse und der vierten Quelle, die zwischen der biologischen Gewebefläche und dem optischen Teiler liegen, gemeinsame Teile sind.

7. Intraoperative optische Sonde nach Anspruch 5 oder 6, **dadurch gekennzeichnet, dass** die Bildverarbeitungsmittel Mittel zum Erkennen der markierten Zonen umfassen, und dadurch, dass die Sonde elektronische Mittel zum Aktivieren der vierten Lichtquelle umfasst, wenn eine markierte Zone von der dritten Lichtquelle beleuchtet wird.

8. Medizinisches Gerät, das mit einer Sonde nach einem der Ansprüche 5 bis 7 verbunden ist und die Verarbeitungsmittel und das Steuergerät der vierten Lichtquelle umfasst.

9. Medizinisches Gerät nach Anspruch 8 mit einem Anzeigegerät zum Visualisieren des Äquivalents des ersten Bildes oder des zweiten Bildes.

10. Medizinisches Gerät nach Anspruch 8 oder 9, **dadurch gekennzeichnet, dass** sie eine Operationslampe umfasst, die mit zu wenigstens der ersten Wellenlänge unterschiedlichen Wellenlängen emittiert.
